(19) Europäisches Patentamt
European Patent Office
Office européen des brevets

(11) EP 0 353 124 B1

(12) **FASCICULE DE BREVET EUROPEEN**

(45) Date de publication et mention de la délivrance du brevet: **06.03.1996 Bulletin 1996/10**

(51) Int Cl.[6]: **C07H 21/04**, C12Q 1/68

(21) Numéro de dépôt: **89402007.2**

(22) Date de dépôt: **12.07.1989**

(54) **Procédé de marquage d'une sonde nucléique et trousse de réactifs pour la mise en oeuvre de ce procédé**

Verfahren zur Markierung einer Nucleinsäure-Sonde und Reagenzienkit zur Ausführung dieses Verfahrens

Process for labelling a nucleic-acid probe and set of reagents for carrying out the process

(84) Etats contractants désignés:
**AT BE CH DE ES FR GB GR IT LI LU NL SE**

(30) Priorité: **13.07.1988 FR 8809598**
**10.03.1989 FR 8903192**

(43) Date de publication de la demande:
**31.01.1990 Bulletin 1990/05**

(73) Titulaire: **BIOPROBE SYSTEMS**
**F-75012 Paris (FR)**

(72) Inventeur: **Lebacq, Philippe**
**F-75012 Paris (FR)**

(74) Mandataire: **Plaçais, Jean-Yves et al**
**Cabinet Netter,**
**40, rue Vignon**
**F-75009 Paris (FR)**

(56) Documents cités:
**EP-A- 0 124 221**
**EP-A- 0 128 332**
**EP-A- 0 131 830**
**EP-A- 0 138 357**
**EP-A- 0 151 001**
**EP-A- 0 173 251**
**EP-A- 0 185 547**
**WO-A-87/02667**
**WO-A-87/03622**

- **CHEMICAL ABSTRACTS, vol. 94, 1981, page 256, no. 60280s, Columbus, Ohio, US; S. M. POLITZ et al.: "Ribonucleic acid-protein cross-linking in Escherichia coli ribosomes: (4-azidophenyl) glyoxal, a novel heterobifunctional reagent", & BIOCHEMISTRY 1981, 20(2), 372-8**
- **CHEMICAL ABSTRACTS, vol. 107, 1987, page 299, no. 129513b, Columbus, Ohio, US; M. OSSWALD et al.: "RNA-protein crosslinking in Escherichia coli 30S ribosomal subunits; determination of sites on 16S RNA that are crosslinked to proteins S3, S4, S5, S7, S8, S9, S11, S13, S19 and S21 by treatment with methyl p- azidophenyl acetimidate", & NUCLEIC ACIDS RES. 1987, 15(8), 3221-40**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).



Printed by Jouve, 75001 PARIS (FR)

## Description

L'invention concerne un procédé de marquage non radioactif d'une sonde nucléique, ainsi qu'une trousse de réactifs pour la mise en oeuvre de ce procédé.

Les sondes nucléiques constituent une technologie récente pour la détection et l'identification de séquences génétiques spécifiques aux êtres biologiques dans des échantillons soumis à analyse. Elles sont utilisées pour la détection des acides nucléiques en particulier ceux de micro-organismes, tels que les virus ou les bactéries dans le domaine médical, vétérinaire ou agro-alimentaire.

Une sonde nucléique comprend, pour l'essentiel, une séquence nucléique ou "séquence-sonde", à savoir une séquence d'ARN ou d'ADN à simple brin qui est capable, dans des conditions expérimentales définies, de retrouver sa séquence nucléique complémentaire ou "séquence-cible", si cette dernière est présente dans l'échantillon biologique soumis à analyse et mis au contact de la sonde.

En pareil cas, la séquence nucléique-sonde et la séquence nucléique-cible s'hybrident, en vertu de la complémentarité de leurs structures, pour former un système stable appelé "duplex".

Pour révéler la présence de l'hybride, et par conséquent celle de la séquence nucléique-cible recherchée, il faut marquer la sonde afin d'obtenir un signal de détection sur le site même de la réaction d'hybridation.

On a utilisé, pour réaliser ce marquage, des sondes radioactives permettant de détecter l'hybridation par comptage ou auto-radiographie.

Pour éviter les inconvénients inhérents à la manipulation d'isotopes radioactifs, des sondes nucléiques dites "froides", ne comportant pas d'éléments radioactifs, ont été développées ces dernières années.

Il existe ainsi différents procédés permettant de détecter des hybrides nucléiques sans faire appel à des isotopes radioactifs.

Les premiers procédés, de type non radioactif, qui ont été développés, ont fait appel à des systèmes de détection indirecte où un nucléotide modifié a été incorporé dans la séquence nucléique-cible par synthèse enzymatique in vitro. Le système le plus courant utilise des nucléotides biotinylés, c'est-à-dire des nucléotides comportant un motif biotine. Ces nucléotides sont incorporés à la séquence nucléique-sonde par la méthode enzymatique dite de "déplacement de coupure" (nick translation en anglais). La séquence nucléique-sonde biotinylée est hybridée à la séquence nucléique-cible (ADN ou ARN). L'hybride ainsi formé est ensuite reconnu par une protéine possédant une très forte affinité pour la biotine : par exemple de l'avidine, protéine isolée du jaune d'oeuf, ou encore de la streptavidine, protéine isolée de certains streptomyces. Pour donner une idée de cette affinité, on peut signaler que la constante de dissociation Kd de la streptavidine pour la biotine est égale à $10^{-15}$ M. Cette protéine (avidine ou streptavidine) peut être directement couplée à une molécule de phosphatase alcaline ou bien être ensuite elle-même reconnue par un complexe de phosphatases alcalines biotinylées. Par la suite, la phosphatase alcaline peut hydrolyser un substrat incolore et soluble en un produit coloré qui précipite in situ, ce qui permet de détecter l'hybride sur le lieu même de la réaction.

Malheureusement, la sensibilité des systèmes avidine-biotidine, ou streptavidine-biotine, n'atteint que difficilement celle des systèmes radioactifs. En outre, ces systèmes peuvent générer un bruit de fond important dans certaines applications et leur mise en oeuvre, assez lourde, est rédhibitoire pour une utilisation en routine dans des laboratoires d'analyses.

D'autres procédés de marquage indirect de sondes nucléiques ont été développés, dont certains font appel à une modification chimique de la séquence nucléique-sonde. La modification, de nature antigénique, apporte à la séquence-sonde des épitopes spécifiques susceptibles d'être reconnus par des anticorps. Après fixation sur l'épitope, ces anticorps, le plus souvent monoclonaux, sont ensuite reconnus par des anticorps polyclonaux liés par covalence à des enzymes de révélation, par exemple phosphatase alcaline, peroxydase, glucosidase, β-galactosidase, etc. Ces procédés, quoique très sensibles, sont encore d'un emploi compliqué et sont, par conséquent, réservés à des laboratoires de recherche fondamentale ou à d'importants laboratoires de recherche appliquée. Leur mise en oeuvre est encore trop longue et trop contraignante pour les laboratoires de routine diagnostique.

En dehors des procédés de marquage indirect, des procédés de marquage direct ont été réalisés récemment. Le marquage se fait alors par incorporation chimique d'un nucléotide modifié sur lequel on a greffé une enzyme de révélation telle que la phosphatase alcaline. L'incorporation du nucléotide modifié se fait à l'aide d'un synthétiseur d'oligonucléotides et par couplage chimique. L'un des inconvénients de ces procédés de marquage direct réside dans le fait que cette synthèse nécessite obligatoirement de connaître la séquence de l'ADN cible que l'on recherche. Au surplus, le prix et la complexité de la séquence ne permettent d'obtenir que de courtes séquences nucléiques (oligonucléotides) sur lesquelles il n'est possible de greffer qu'une seule molécule d'enzyme par molécule d'oligonucléotide sous peine de déstabiliser l'hybride formé. Ce rapport mole à mole diminue la sensibilité de ce type de sonde.

Enfin, avec ces procédés de marquage direct, il n'est guère possible d'envisager un marquage sans posséder un synthétiseur d'ADN.

Les différentes contraintes énoncées plus haut pour ce dernier procédé limitent d'autant :

- la possibilité d'utiliser des sondes longues pour les études de RFLP (Restriction Fragment Length Polymorphism) ce qui signifie : polymorphisme de longueur des fragments de restriction ;

- la possibilité d'utiliser des sondes dont la séquence nucléique-sonde n'est pas connue ou seulement partiellement connue ;

- la possibilité d'utiliser directement un acide nucléique naturel ou un acide nucléique cloné.

On connaît aussi, d'après EP-A-0 128 332, un procédé de marquage d'acide nucléique dans une sonde nucléique comportant une séquence nucléique-sonde propre à s'hybrider avec une séquence nucléique-cible complémentaire, et dans lequel on réalise le marquage dudit acide nucléique par pontage chimique direct d'un système de révélation pour permettre une détection ultérieure de l'hybride ainsi formé.

Il s'agit toutefois d'un procédé de marquage spécifique qui met en oeuvre deux phages M13 et qui est lourd à mettre en oeuvre.

Par ailleurs, EP-A-0 151 001 décrit des sondes comprenant une séquence nucléique-sonde et une séquence modifiée qui contient au moins un système de révélation.

En outre, EP-A-0 124 221 décrit des sondes comprenant une séquence nucléique-sonde et un acide nucléique simple-brin qui porte comme système de révélation une enzyme, les deux séquence étant liées par hybridation.

C'est, en conséquence, l'un des buts de l'invention de procurer un procédé de marquage non radioactif d'une sonde nucléique qui permet d'éviter les inconvénients des procédés de marquage connus dans la technique antérieure. C'est, en particulier, un but de l'invention de procurer un procédé de marquage permettant l'utilisation de sondes courtes ou longues, de séquences connues ou inconnues, sans nécessiter l'utilisation d'un synthétiseur à ADN.

C'est aussi un but de l'invention de procurer un tel procédé de marquage qui soit adapté à la détection d'acides nucléiques, comme les acides nucléiques viraux ou bactériens ou plus généralement ceux des micro-organismes dont la détection est importante dans le domaine médical, vétérinaire ou agro-alimentaire.

C'est encore un but de l'invention de procurer un tel procédé de marquage qui soit utilisable pour la détection des maladies génétiques ou l'analyse de cellules tumorales dans un but diagnostique, pronostique ou thérapeutique.

C'est également un but de l'invention de procurer un tel procédé de marquage qui soit propice à la fabrication de trousses de réactifs diagnostiques ou "kits" comme à une utilisation propre à la recherche fondamentale dans laquelle le chercheur marque lui-même ses sondes, quel qu'en soit le type.

C'est encore un but de l'invention de procurer un tel procédé de marquage qui permet une amplification du signal de détection par rapport aux procédés et systèmes connus jusqu'à présent.

Selon un premier aspect, l'invention concerne un procédé de marquage d'un acide nucléique dans une sonde nucléique comportant une séquence nucléique-sonde propre à s'hybrider avec une séquence nucléique-cible complémentaire, et dans lequel on réalise le marquage dudit acide nucléique par pontage chimique direct d'un système de révélation, en particulier d'une enzyme de révélation, pour permettre une détection ultérieure de l'hybride ainsi formé.

Conformément à l'invention, on réalise ce marquage par pontage chimique direct du système de révélation sur les groupements Guanine de l'acide nucléique de la sonde au moyen d'un composé hétérobifonctionnel choisi parmi le 4-azidophényl-glyoxal (APG) et le kéthoxal ou leurs dérivés, ledit acide nucléique de la sonde étant un acide nucléique simple-brin modifié qui est incapable par lui-même de s'hybrider à la séquence nucléique-cible et qui est accroche à la séquence nucléique-sonde propre à s'hybrider par liaison chimique covalente.

Ainsi, le pontage chimique du système de révélation ne s'effectue pas sur la séquence nucléique-sonde proprement dite, mais sur un acide nucléique modifié qui est incapable de s'hybrider par lui-même sur la séquence nucléique-cible et qui est lui-même relié à cette séquence nucléique-sonde.

Le composé hétérobifonctionnel utilisé dans le procédé de l'invention est capable de réagir à la fois avec le ou les acides nucléiques à marquer et un système de révélation qui peut être notamment une protéine, ce qui permet d'établir un pontage entre les deux types de molécules. Ce pontage permet ainsi de lier le système de détection, par exemple une enzyme ou tout autre composé, directement sur la sonde nucléique, et cela quelle que soit la longueur et quelle que soit la nature (ADN ou ARN) de la sonde proprement dite, c'est-à-dire de sa séquence nucléique-sonde.

Le procédé de l'invention peut s'appliquer à la fois au diagnostic de routine par la réalisation de trousses de réactifs (kits) de détection d'acides nucléiques de micro-organismes, au diagnostic de certaines maladies génétiques et à une utilisation à des fins de recherche fondamentale où le chercheur possède ses propres sondes qu'il peut marquer lui-même.

De façon générale, le pontage du système de révélation s'effectue sur les Groupements Guanine de l'acide nucléique à marquer, de sorte que l'acide nucléique ainsi modifié soit incapable de s'hybrider ultérieurement avec un acide nucléique-cible, la formation totale ou partielle d'un hybride entre la sonde et la cible étant impossible sous quelque condition d'hybridation que ce soit.

Les composés hétérobifonctionnels utilisés dans l'invention sont des composés chimiques possédant d'une part

une fonction propre à établir une liaison avec l'acide nucléique à marquer et, d'autre part, une fonction propre à établir une liaison avec le système de révélation choisi.

Les composés hétérobifonctionnels utilisés dans l'invention sont choisis parmi le 4-azidophényl-glyoxal (APG) répondant à la formule générale :

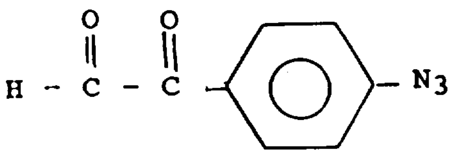

le kéthoxal répondant à la formule générale :

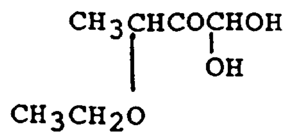

et leurs dérivés.

L'APG se fixe au niveau de l'amine portée par le carbone 2 et au niveau de l'azote 1 de la Guanine, cette amine et cet azote permettant habituellement la formation d'une liaison hydrogène, respectivement avec l'oxygène de la fonction cétone en $C_2$ et avec l'azote 3 de la cytosine lors de la formation d'hybrides nucléiques. La fixation de l'APG empêche alors la formation d'une telle liaison hydrogène.

Le kéthoxal et le 4-azidophényl-glyoxal (APG) ont été décrits dès 1969 comme capables de se fixer spécifiquement sur les résidus Guanine non appariés des ARN de transfert conférant à ces derniers une résistance aux attaques par la RNase TI.

Ces composés hétérobifonctionnels ont été utilisés dans la littérature pour réaliser des pontages entre protéines et ARN ribosomiques des particules 30S des ribosomes d'E. Coli et pour étudier ainsi les sites de contact entre les protéines et l'ARN qui constituent les ribosomes.

Par conséquent, les composés hétérobifonctionnels utilisés dans la mise en oeuvre du procédé de l'invention ont une fonction et permettent d'aboutir à des résultats totalement différents de ceux qu'ils avaient remplis jusqu'à présent.

En effet, conformément à l'invention, la fonction du composé hétérobifonctionnel est de réaliser un couplage entre un système de révélation, notamment une enzyme, et l'acide nucléique à marquer qui ne fait pas partie de la sonde.

Dans le cas où le composé hétérobifonctionnel utilisé dans l'invention est du type comportant une fonction dicarbonyle et une fonction phényl-azide, on fait d'abord réagir le composé hétérobifonctionnel, dans l'obscurité, avec l'acide nucléique à ponter, de manière à former une liaison covalente entre le composé hétérobifonctionnel et les groupements Guanine de l'acide nucléique. Ensuite, on fait réagir l'ensemble sous photo-activation, avec un système de révélation propre à former une liaison avec le nitrène qui résulte de la photo-activation de la fonction azide.

On réalise le pontage du système de révélation sur un acide nucléique ne faisant pas partie de la sonde et on accroche ensuite l'acide nucléique ponté à la sonde. Dans ce cas, l'acide nucléique sur lequel le pontage a été réalisé est ensuite lui-même lié à l'acide nucléique sonde, c'est-à-dire à la séquence sonde, par une liaison phospho-ester faisant intervenir une ligase ou bien par un procédé de couplage chimique.

En effet, les composés hétérobifonctionnels utilisés, notamment l'APG, réagissent avec les bases aromatiques des acides nucléiques. L'APG réagit avec les groupements aminés qui permettent aux liaisons hydrogène de s'établir entre la Guanine et la Cytosine dans les duplex d'acides nucléiques. Ainsi, lorsque l'APG est fixé sur une Guanine, celle-ci ne peut plus établir ensuite de liaison hydrogène avec une Cytosine. Par ailleurs, après couplage avec le système de révélation, par exemple une molécule de phosphatase alcaline, dont la masse moléculaire est de l'ordre de 80.000 à 120.000 daltons, l'encombrement stérique apporté par la protéine conduit à un non-appariement sur une longueur moyenne de 250 nucléotides environ par molécule d'enzyme fixée.

L'acide nucléique accroché à la sonde est un acide nucléique non codant, naturel ou synthétique, choisi parmi de l'ADN, de l'ARN, du poly-A-G (formé uniquement de bases Adénine et Guanine) et de poly-dA-dG, ou bien un acide nucléique naturel dont la séquence est choisie de manière à ne pas interférer avec la séquence nucléique-cible pendant l'hybridation.

On préfère tout particulièrement utiliser des séquences statistiques ou "randomisées" d'acide nucléique simple brin comme le poly-A-G ou le poly-dA-dG et de longueur variable, ce qui donne tout loisir pour fixer un grand nombre de molécules du système de révélation, notamment d'une enzyme de révélation. La sensibilité de la détection s'en trouve améliorée et il n'y a pas à craindre de fixation non spécifique de ces séquences.

Conformément à l'invention, la réaction de pontage doit s'effectuer sur un acide nucléique préalablement dénaturé, c'est-à-dire du type monobrin.

Le système de révélation est de préférence une protéine choisie notamment parmi des enzymes, en particulier la phosphatase alcaline, des anticorps, etc.

Si le système de révélation est constitué par une protéine, on peut former ensuite sur cette protéine un complexe protéique, enzymatique ou multi-enzymatique propre à amplifier le signal de détection. La formation du complexe peut s'effectuer par des méthodes connues, en particulier en utilisant du glutaraldéhyde ou encore du N-éthoxyl-1,2-dihydroquinoline (EEDQ) pour former des liaisons covalentes protéine-protéine.

On obtient ainsi une amplification élevée de la détection, étant donné que, d'une part, plusieurs moles de système de détection sont liés à la sonde par mole de cette dernière et que, d'autre part, on peut greffer ensuite des complexes générant des signaux amplifiés.

Dans une autre variante, le système de révélation est de nature non protéique et choisi notamment parmi des composés fluorescents ou des composés phosphorescents.

Ainsi, conformément à l'invention, la partie propre de la sonde, c'est-à-dire la séquence nucléique-sonde complémentaire de la séquence nucléique cible recherchée dans le milieu biologique ou dans l'analyse, peut avoir une longueur importante ou, au contraire, être très courte au gré de l'expérimentateur. Cette souplesse permet une utilisation universelle de ce type de marquage.

En effet, les séquences longues pourront être utilisées pour réaliser des hybridations utilisant des acides nucléiques clonés de séquences généralement importantes. Ces acides nucléiques clonés peuvent être obtenus facilement et en grande quantité par les techniques d'amplification plasmidique. De plus, il n'est pas nécessaire d'en connaître la séquence pour les utiliser. Ce type de sonde est particulièrement utile pour les études de RFLP.

D'autre part, les séquences courtes ou oligonucléotidiques, c'est-à-dire comprises entre 15 et 50 nucléotides, peuvent être employées de la même manière en utilisant ce type de marquage. L'intérêt des sondes oligonucléotidiques réside dans la vitesse à laquelle l'hybridation peut être réalisée. Ainsi, le temps (t ½) au bout duquel la moitié d'un ADN est renaturée est égal à 2,5 minutes avec une sonde de 15 nucléotides de longueur à une concentration de l'ordre de 10 nM. Dans les mêmes conditions, il faudrait entre 6 et 24 heures pour une sonde de longueur classique, c'est-à-dire de 1.000 à 5.000 nucléotides. Cet important gain de temps est vital pour permettre à la technologie des sondes non-radioactives d'être utilisée en routine dans les laboratoires d'analyses diagnostiques privés ou publics.

Sous un autre aspect, l'invention concerne un ensemble de réactifs ou "kit" pour la mise en oeuvre du procédé défini plus haut.

Cet ensemble comprend, pour l'essentiel :

- un flacon d'acides nucléiques pontés avec un système de détection, par exemple une enzyme de détection ;
- un flacon d'un système de ligation, par exemple une enzyme de ligation ;
- un flacon d'un mélange réactionnel tamponné, propre à être mélangé aux substrats (acide nucléique-sonde et acide nucléique-ponté au système de détection);
- un flacon d'une solution tampon, propre à être mélangée à au moins un substrat chromogène, propre à réagir avec le système de révélation ; et
- un flacon d'au moins un substrat chromogène.

Dans une autre forme de réalisation de l'invention, l'acide nucléique marqué est un vecteur simple brin comportant une séquence nucléique circulaire.

L'avantage d'utiliser un tel vecteur réside notamment dans la possibilité de ponter le système de révélation tout autour de la séquence circulaire, par l'intermédiaire du composé hétérobifonctionnel, et d'amplifier ainsi le signal de révélation. Un autre avantage qui en résulte est de ne pas avoir à dénaturer cet acide nucléique et de ne pas craindre une auto-renaturation dans le milieu d'hybridation.

Le vecteur simple brin est avantageusement un acide nucléique d'un phage, d'un virus, d'un plasmide ou encore d'un épisome.

Le pontage du système de révélation sur ledit vecteur est nécessairement réalisé sur les groupements Guanine ou Thymine à l'aide d'un composé hétérobifonctionnel de telle manière que la séquence du vecteur ne puisse plus jamais s'hybrider. On évite ainsi les hybridations indésirables que l'on peut obtenir avec certains vecteurs, comme le phage M13 qui s'hybride avec certaines séquences du génome humain.

On préfère utiliser un phage de type M13 car il s'agit d'un phage très utilisé classiquement en biologie moléculaire. Les phages M13 sont des bactériophages filamenteux dont le génome est constitué d'un ADN simple brin circulaire de 6500 nucléotides environ et ils sont utilisés habituellement comme vecteurs de séquençage. Mais on peut aussi utiliser tout autre bactériophage de ce type, par exemple le phage Fd,ϕQx 174, G 4, R 17, etc.

Dans une variante de réalisation, le vecteur simple brin comporte, clonée dans sa séquence circulaire, la séquence nucléique-sonde.

On obtient ainsi une sonde spécifique qui est prévue pour la détection d'une séquence nucléique-cible complémentaire de la séquence nucléique-sonde.

Par exemple, la séquence nucléique-cible clonée dans le vecteur simple brin peut être celle du virus HIV du SIDA, ce qui permet d'obtenir une sonde de détection spécifique de ce virus.

La séquence à insérer doit être clonée dans les deux sens de telle manière que chaque séquence complémentaire simple-brin soit dans deux vecteurs simple-brin distincts. Le marquage s'effectue après hybridation des deux séquences complémentaires clonées. Les séquences des deux vecteurs simple-brin étant identiques, elles ne s'hybrident pas et subissent par conséquent le pontage chimique. On sépare ensuite les deux séquences clonées complémentaires, préalablement hybridées, par traitement à l'urée. Ces deux séquences sont libres de tout pontage, contrairement au vecteur qui les porte et peuvent s'hybrider à la séquence nucléique-cible ultérieurement.

Cette variante de réalisation est donc tout particulièrement intéressante à des fins de diagnostic, du fait de la spécificité de la sonde. La détection de la séquence nucléique-cible éventuellement présente dans l'échantillon biologique à analyser implique alors une seule hybridation, c'est-à-dire l'hybridation entre la séquence nucléique-cible et la séquence nucléique-sonde.

Les systèmes de révélation utilisables peuvent être des protéines, notamment des enzymes telles que la phosphatase alcaline, des anticorps, etc., ou encore des systèmes de nature non protéique, notamment des composés fluorescents ou phosphorescents.

Selon un autre aspect, l'invention préconise d'utiliser dans le procédé de marquage un autre système de révélation.

Le système envisagé est un système de bio-luminescence, c'est-à-dire un système à la fois protéique et luminescent, qui est propre à transformer un substrat non luminescent en un produit luminescent.

Comme systèmes de bio-luminescence utilisables conformément à l'invention, on peut citer notamment les systèmes utilisant la luciférase et la luciférine selon la réaction suivante :

$$LH_2 + ATP + O_2 \xrightarrow{M^{++}} L=O + CO_2 + AMP + PP_i + h\nu \ .$$

Une autre réaction peut être utilisée dans laquelle le système luminescent consiste en deux catalyses :

- l'une avec une NAD(P)H : FMN oxydoréductase,
- l'autre avec une luciférase bactérienne selon les réactions suivantes :

$$NAD(P)H + FMN + H^+ \rightarrow NAD(P)^+ + FMNH_2$$

$$FMNH_2 + RCHO + O_2 \rightarrow FMN + RCOOH + H_2O + h\nu$$

Par ailleurs, sous un autre aspect, l'invention concerne aussi une trousse de réactifs, ou "kit", pour la mise en oeuvre du procédé défini précédemment.

Cet ensemble comprend essentiellement :

- 1 flacon contenant de la luciférine
- 1 flacon contenant de l'ATP et un tampon réactionnel
- 1 flacon contenant de la luciférase

ou bien

- 1 flacon contenant un composé aldéhydique à longue chaîne,
- 1 flacon contenant du NAD(P)H + FMN + $H^+$ + un tampon réactionnel
- 1 flacon contenant une diaphorase
- 1 flacon contenant de la luciférase bactérienne

ou encore

- 1 flacon contenant un composé aldéhydique à longue chaîne
- 1 flacon contenant du $FMNH_2$ et un tampon réactionnel
- 1 flacon contenant de la luciférase bactérienne.

Dans la description qui suit, donnée seulement à titre d'exemple, on se réfère aux dessins annexés, sur lesquels :

- la figure 1 représente schématiquement une sonde nucléique selon l'invention dans une première variante,
- la figure 2 représente schématiquement une sonde nucléique dans une seconde variante,
- la figure 3 représente schématiquement une sonde nucléique selon l'invention dans une troisième variante,
- les figures 4A à 4E représentent schématiquement les étapes successives de clonage et de marquage d'une séquence dans un vecteur simple-brin.

Sur la figure 1, on a représenté schématiquement une séquence nucléique-cible SNC fixée sur un support solide approprié, par exemple sur une membrane M de nylon ou de nitrocellulose.

La séquence nucléique-cible, qui peut être par exemple un ADN monobrin, est propre à s'hybrider avec une séquence nucléique-sonde SNS monobrin et de structure complémentaire pour former un hybride.

La séquence nucléique-sonde SNS fait partie d'une sonde conformément à l'invention. A chaque extrémité de la séquence SNS est accroché un acide nucléique marqué ANM, le marquage étant effectué conformément à l'invention.

Ainsi, des molécules d'un composé hétérobifonctionnel CHB sont liées, d'une part, à l'acide nucléique marqué et, d'autre part, à un système de révélation, dans l'exemple une molécule de phosphatase alcaline PA jouant le rôle de système de détection. La phosphatase alcaline est capable de réagir avec un substrat chromogénique SC incolore et de catalyser sa transformation en un produit coloré et insoluble qui précipite in situ, c'est-à-dire sur le lieu même de la réaction.

A titre d'exemple, on utilise l'APG en tant que composé hétérobifonctionnel pour réaliser le pontage chimique direct du système de révélation sur l'acide nucléique à marquer. Cet acide nucléique est dénaturé s'il est sous forme double-brin par chauffage à 100°C pendant 7 minutes, puis refroidi brutalement dans la glace à 0°C pour éviter toute renaturation.

L'APG est un alpha-dicarbonyle qui est propre à réagir avec les groupements Guanidinium de l'acide nucléique devant être marqué. La réaction est plus spécifique des résidus Guanine à pH neutre et en présence de borate. Cette première réaction doit nécessairement être réalisée à l'obscurité pour éviter la photolyse de la fonction azide.

La première réaction qui conduit à la liaison covalente APG-Guanine a lieu dans un tampon : 0,1 M acide 3-(N-morpholino)-2-propanesulfonique ou MOPS, pH : 7,0, 0,02 M borate de sodium, 0,01 M MgCl2. Pour 10 à 20 microgrammes d'acide nucléique simple brin en solution dans ce tampon, on ajoute de l'APG à l'obscurité à partir d'une solution stock congelée à 200 mg par ml dans le dioxanne de manière que la concentration finale en APG soit de 1,5 mg par ml. La réaction a lieu à l'obscurité pendant 30 minutes à 37°C. Les acides nucléiques sont ensuite précipités à l'obscurité avec de l'éthanol absolu à -20°C. Le précipité est ensuite centrifugé et remis en suspension dans le même tampon MOPS sans APG.

La deuxième réaction consiste à réaliser une liaison covalente entre l'APG lié à l'acide nucléique et une protéine ou tout composé chimique susceptible de réaliser une liaison avec un nitrène. Les aryles-azides, inactifs dans l'obscurité, forment des aryles-nitrènes lorsqu'ils sont photoactivés. Ces groupes sont très instables et réagissent avec les protéines pour donner des liaisons covalentes.

A la solution précédente, on ajoute le système de détection que l'on désire coupler.

Dans cet exemple, ce système de détection est une enzyme, à savoir la phosphatase alcaline, que l'on ajoute à une concentration finale de 120 microgrammes par ml.

Le mélange est placé dans une cuve de quartz de 1 à 2 mm d'épaisseur et irradié pendant 15 à 45 minutes sous une lampe à ultraviolets de faible puissance. La cuve est directement placée contre la lampe avec, cependant, un couvercle de boîte de Pétri en pyrex, intercalé entre la lampe et la cuve. Le pyrex permet de filtrer les rayonnements ultraviolets de longueur d'onde inférieure à 300 nM.

Après irradiation, le mélange est directement placé sur une colonne d'Ultrogel AcA-34 afin de séparer et de purifier les acides nucléiques couplés à la phosphatase alcaline qui viennent d'être préparés.

Les fractions utiles récupérées sont rassemblées et précipitées avec de l'éthanol absolu à -20°C.

La sensibilité demandée le plus souvent doit permettre la détection d'une séquence unique dans un génome ou la détection d'ARN messagers rares. L'invention se prête tout à fait à ces exigences. Dans ce cas, le pontage s'effectue sur un acide nucléique non-codant préparé, par exemple, par synthèse enzymatique ou sur un acide nucléique naturel dont la séquence n'interférera pas avec la séquence nucléique-cible au cours de l'hybridation.

L'exemple qui suit illustre l'accrochage à la séquence nucléique-sonde de l'acide nucléique préalablement ponté.

Dans une forme de réalisation préférée de l'invention, les acides nucléiques non-codants sont constitués de poly-A-G ou de poly-dA-dG simple brin dont la séquence est obtenue au hasard. Ces acides nucléiques peuvent être réalisés par l'utilisateur au laboratoire ou achetés facilement dans le commerce. Le pontage avec des molécules de système de révélation, par exemple de phosphatase alcaline, est réalisé à l'aide de l'APG.

L'acide nucléique, lié de façon covalente à l'enzyme, est ensuite lui-même lié ou accroché à la sonde proprement dite, c'est-à-dire à la séquence nucléique-sonde, par une réaction de ligation faisant intervenir de l'ARN ligase. Il se produit ainsi une liaison alcool-acide qui donne un phospho-ester qui permet de relier les deux acides nucléiques.

Le pontage des acides nucléiques faisant intervenir l'APG et ses dérivés nécessite la dénaturation préalable des acides nucléiques. Tous les acides nucléiques utilisés sont donc simple brin. Dans une réalisation préférée de l'invention, les acides nucléiques pontés sont liés à la sonde (séquence nucléique-sonde) en utilisant de l'ARN ligase. Cette enzyme est en effet capable de réaliser la formation d'une liaison phospho-diester entre deux acides nucléiques simple brin ADN ou ARN. Son activité est particulièrement élevée vis-à-vis de l'ARN.

A titre d'exemple, la ligation est réalisée pendant la nuit à 37°C en présence de 4 microgrammes de T4 ARN ligase dans un tampon contenant 0,05 M Tris HCl pH 7,6, 0,01 M MgCl2, 0,01 M adénosine-triphosphate (ATP), 0,01 M dithiothréitol, 5 % polyéthylèneglycol 8000 (PEG 8000) en concentration finale et 2 microgrammes d'acide nucléique totaux. Le rapport acide nucléique-sonde/acide nucléique-ponté est généralement de 1/3 à 1/5 en termes de concentration d'extrémité.

Comme indiqué plus haut, le nombre de molécules d'enzyme, ou plus généralement de molécules du système de détection, fixé par molécule de sonde, peut être augmenté de façon importante. Cette augmentation est possible car elle ne nuit pas à la stabilité future des hybrides qui se forment puisque les nucléotides pontés n'interviennent pas dans l'hybridation elle-même.

L'augmentation du nombre de molécules d'enzyme (ou autre système de détection) liées par molécule de sonde est obtenue par liaison covalente protéine-protéine à l'aide de la glutaraldéhyde ou de l'EEDQ selon des méthodes classiques connues. Ainsi, on peut aboutir, par exemple, à des complexes de poly-phosphatases alcalines. On peut prévoir de réaliser également la fixation de complexes multi-enzymatiques à plusieurs substrats capables d'augmenter encore l'importance du signal généré par la sonde hybridée ou tout autre système propre à générer un signal intéressant et exploitable.

Le procédé de l'invention trouve ainsi une application universelle, c'est-à-dire qu'il peut être utilisé pour marquer tous les types de sondes pour chaque application spécifique. Ce procédé de marquage peut être appliqué aussi bien au diagnostique rapide avec des oligonucléotides que pour des applications de recherche fondamentale ou encore pour l'étude des RFLP ou les sondes comportent souvent des séquences longues.

Le procédé de l'invention est très sensible et plus facile à mettre en oeuvre que les autres procédés existants puisqu'il ne fait pas intervenir de molécules intermédiaires, telles que protéines et leurs ligands, systèmes anticorps-antigènes, etc. La révélation de l'hybride nucléique est donc immédiate après hybridation.

Le procédé permet également l'utilisation de complexes multi-enzymatiques ou de tout autre système biologique, biochimique ou chimique, propre à augmenter efficacement le signal de détection sans nuire à la réalisation et à la stabilité de l'hybride nucléique.

La très bonne sensibilité du marquage permet l'utilisation de sondes plus courtes. Ces sondes plus courtes limitent ainsi la complexité de la renaturation et donc le temps de la réaction. Les temps d'hybridation sont ainsi considérablement réduits.

A titre d'exemple, l'ensemble de réactifs pour la mise en oeuvre du procédé peut comprendre les éléments suivants :

- un flacon d'acides nucléiques pontés avec la phosphatase alcaline ;
- un flacon d'une enzyme de ligation (par exemple ARN ligase) ;
- un flacon d'un mélange réactionnel tamponné, par exemple Tris acétate 100 mM, pH 9,4, acétate de sodium 100 mM, acétate de magnésium 10 mM ;
- un flacon d'une solution tampon propre à être mélangée aux substrats chromogène de l'enzyme de révélation ;
- un flacon d'un composé chromogénique n° 1, par exemple 5-bromo-4-chloro-3-indolyl-phosphate ;
- un flacon d'un composé chromogénique n° 2, par exemple nitrobleu de tétrazolium.

Sur la figure 2 à laquelle on se réfère maintenant, on a représenté schématiquement une séquence nucléique-cible SNC faisant partie d'un échantillon biologique à analyser.

La séquence nucléique-cible, qui peut être par exemple un ADN monobrin, est fixée sur un support solide approprié (non représenté), par exemple une membrane de nylon ou de nitrocellulose.

Pour détecter la séquence nucléique-cible, on fait appel à une sonde comprenant une séquence nucléique-sonde SNS mono-brin, de structure complémentaire de la séquence SNC, et propre à s'hybrider à celle-ci. Sur la séquence nucléique-sonde SNS est accrochée une séquence nucléique comportant une partie du gène de résistance à l'Ampicilline, désigné par $Amp^R$. Cette partie de gène $Amp^R$ est propre à s'hybrider avec une autre partie de gène $Amp^R$ intégrée dans la séquence nucléique circulaire monobrin d'un phage M13. La partie du gène $Amp^R$ est clonée, par des techniques de clonage en elles-mêmes connues, au sein de la séquence circulaire du phage M13.

Sur la séquence nucléique circulaire du phage M13 est ponté chimiquement, au moyen d'un composé hétérobifonctionnel CHB, un système de révélation SR pouvant être constitué notamment par une enzyme de révélation. Le pontage chimique du système de révélation sur la séquence nucléique du phage M13 s'effectue conformément à la technique décrite plus haut.

Le vecteur M13 peut ainsi s'hybrider avec la séquence nucléique-sonde, et cela par l'intermédiaire des deux parties de gène $Amp^R$. Le vecteur M13 marqué et incorporant le gène $Amp^R$ est ainsi d'une application universelle puisqu'il peut s'hybrider avec toute sonde comportant une séquence nucléique-sonde SNS quelconque, du moment que celle-ci comporte le gène $Amp^R$.

Ainsi, la détection de la séquence nucléique SNC, éventuellement présente dans l'échantillon à tester, s'effectue par une double hybridation. Ces deux hybridations peuvent être effectuées en succession : on hybride d'abord le vecteur M13 sur la sonde et on hybride ensuite la séquence nucléique-sonde SNS avec la séquence nucléique-cible SNC. On peut aussi effectuer simultanément l'hybridation du vecteur M13 avec la sonde et l'hybridation de la séquence SNS avec la séquence SNC. Dans tous les cas, ces hybridations s'effectuent avantageusement en milieu liquide, ainsi qu'il est bien connu dans cette technique.

Dans la forme de réalisation de la figure 3, à laquelle on se réfère maintenant, la séquence nucléique-cible SNC à analyser est détectée au moyen d'une sonde spécifique.

Ladite sonde comprend une séquence nucléique-sonde SNS, complémentaire de la séquence nucléique-cible SNC à détecter, et clonée dans la séquence nucléique monobrin circulaire d'un phage M13 identique ou analogue au phage décrit précédemment.

Sur la séquence nucléique du phage M13 est également ponté chimiquement, par l'intermédiaire d'un composé hétérobifonctionnel CHB, un système de révélation SR. La détection de la séquence SNC s'effectue alors par simple hybridation. Si la séquence SNS de la sonde reconnaît une séquence SNC complémentaire, ces deux séquences s'hybrideront pour former un duplex et le système de révélation SR se trouvera ainsi lié in situ à la séquence SNC.

Dans les exemples des figures 2 et 3, le système de révélation SR peut être constitué par une enzyme ou par tout autre système de révélation comme décrit plus haut, ou encore par un système de bio-luminescence.

On se réfère maintenant aux figures 4A à 4E illustrant le clonage et le marquage d'une séquence, dans l'exemple celle d'une partie du gène $Amp^R$, dans un vecteur simple-brin, dans l'exemple le phage M13.

La séquence $Amp^R$, qui possède deux brins complémentaires $B_1$ et $B_2$, est clonée dans les deux sens (figure 4A) dans la forme réplicative double-brin du phage M13. Le phage M13 possède une forme réplicative avec un brin + et un brin -. Par ce clonage à double sens, un brin $B_1$ de $Amp^R$ est cloné dans un brin + de M13 et un brin $B_2$ de $Amp^R$ est cloné dans un brin - de M13, et inversement dans l'autre sens.

Après réplication dans une bactérie, on récupère uniquement les brins +, d'où il résulte un phage M13 monobrin incluant le brin $B_1$ et un phage M13 monobrin incluant le brin $B_2$ (figure 4B).

On hybride ensuite les deux phages M13 par les brins $B_1$ et $B_2$ qui sont complémentaires, les deux brins + du vecteur M13 étant identiques et ne pouvant s'hybrider. On obtient ainsi la structure montrée à la figure 4C.

On marque ensuite les séquences (brin +) des deux vecteurs M13 par pontage chimique avec un système de révélation SR par l'intermédiaire d'un composé hétérobifonctionnel CHB (figure 4D).

On sépare ensuite les deux séquences clonées complémentaires par un traitement à l'urée (U) et/ou séparation sur gel et/ou utilisation d'une FPLC et/ou d'une HPLC.

Ceci permet d'obtenir deux séquences $B_1$ et $B_2$, libres de tout pontage, contrairement au vecteur (phage M13 monobrin) qui les porte (figure 4E). Les deux séquences pourront ultérieurement s'hybrider à une séquence cible complémentaire.

## Revendications

1. Procédé de marquage d'un acide nucléique dans une sonde nucléique comportant une séquence nucléique-sonde propre à s'hybrider avec une séquence nucléique-cible complémentaire, et dans lequel on réalise le marquage dudit acide nucléique par pontage chimique direct d'un système de révélation, en particulier d'une enzyme de révélation, pour permettre une détection ultérieure de l'hybride ainsi formé, caractérisé en ce que l'on réalise le marquage par pontage chimique direct du système de révélation sur les groupements Guanine de l'acide nucléique de la sonde au moyen d'un composé hétérobifonctionnel choisi parmi le 4-azidophényl-glyoxal (APG) et le kéthoxal ou leurs dérivés, et en ce que ledit acide nucléique de la sonde est un acide nucléique simple-brin modifié qui est incapable par lui-même de s'hybrider à la séquence nucléique-cible et qui est accroché à la séquence nucléique-sonde propre à s'hybrider par liaison chimique covalente.

2. Procédé selon la revendication 1, caractérisé en ce que l'on fait d'abord réagir le composé hétérobifonctionnel, dans l'obscurité, avec l'acide nucléique à ponter de manière à former une liaison covalente entre le composé hétérobifonctionnel et les groupements Guanine de l'acide nucléique et en ce que l'on fait ensuite réagir l'ensemble, sous photoactivation, avec un système de révélation propre à former une liaison avec le nitrène résultant de la photoactivation de la fonction azide.

3. Procédé selon l'une des revendications 1 et 2, caractérisé en ce que l'acide nucléique accroché à la sonde est un acide nucléique non codant, naturel ou synthétique, choisi parmi de l'ADN, de l'ARN, du poly-A-G, du poly-dA-dG, ou un acide nucléique naturel dont la séquence est choisie de manière à ne pas interférer avec la séquence nucléique-cible pendant l'hybridation.

4. Procédé selon la revendication 3, caractérisé en ce que l'on réalise la réaction de pontage sur un acide nucléique préalablement dénaturé, du type monobrin.

5. Procédé selon l'une des revendications 3 et 4, caractérisé en ce que l'on accroche l'acide nucléique ponté à la sonde par voie chimique.

6. Procédé selon l'une des revendications 1 et 2, caractérisé en ce que ledit acide nucléique marqué est un vecteur simple brin comportant une séquence nucléique simple brin circulaire.

7. Procédé selon la revendication 6, caractérisé en ce que le vecteur simple brin est un acide nucléique d'un phage, d'un virus, d'un plasmide, ou encore d'un épisome.

8. Procédé selon la revendication 7, caractérisé en ce que le vecteur simple brin est un phage de type M13.

9. Procédé selon l'une des revendications 6 à 8, caractérisé en ce que le vecteur comporte, clonée dans sa séquence circulaire, la séquence nucléique-sonde.

10. Procédé selon l'une des revendications 1 à 9, caractérisé en ce que le système de révélation est une protéine choisie notamment parmi des enzymes, en particulier la phosphatase alcaline, des anticorps, etc.

11. Procédé selon la revendication 10, caractérisé en ce que l'on forme ensuite sur la protéine un complexe protéique, enzymatique, ou multi-enzymatique propre à amplifier le signal de détection, la formation du complexe étant effectuée par des méthodes connues, en particulier au moyen de glutaraldéhyde ou de EEDQ pour former des liaisons covalentes protéine-protéine.

12. Procédé selon l'une des revendications 1 à 9, caractérisé en ce que le système de révélation est de nature non protéique et choisi notamment parmi des composés fluorescents, des composés phosphorescents, ou encore un système de bio-luminescence propre à transformer un substrat non luminescent en un produit luminescent.

13. Trousse de réactifs pour la mise en oeuvre du procédé selon l'une des revendications 1 à 12, caractérisé en ce qu'il comprend :

- un flacon d'acides nucléiques pontés avec un système de détection, par exemple, une enzyme de détection ;
- un flacon d'un système de ligation ;
- un flacon d'un mélange réactionnel tamponné propre à être mélangé aux substrats (acide nucléique-sonde et acide nucléique ponté au système de détection) ;
- un flacon d'une solution tampon propre à être mélangée à au moins un substrat chromogène propre à réagir avec le système de révélation ; et
- un flacon d'au moins un susbstrat chromogène.

14. Trousse de réactifs pour la mise en oeuvre du procédé selon l'une des revendications 1 à 12, caractérisée en ce qu'elle comprend essentiellement les composants suivants :

- 1 flacon contenant de la luciférine
- 1 flacon contenant de l'ATP et un tampon réactionnel
- 1 flacon contenant de la luciférase

ou bien

- 1 flacon contenant un composé aldéhydique à longue chaîne,
- 1 flacon contenant du NAD(P)H + FMN + $H^+$ + un tampon réactionnel
- 1 flacon contenant une diaphorase
- 1 flacon contenant de la luciférase bactérienne

ou encore

- 1 flacon contenant un composé aldéhydique à longue chaîne
- 1 flacon contenant du $FMNH_2$ et un tampon réactionnel
- 1 flacon contenant de la luciférase bactérienne.

**Patentansprüche**

1. Verfahren zur Markierung einer Nukleinsäure in einer Nukleinsäure-Sonde mit einer Sonden-Nukleinsäuresequenz, die sich zur Hybridisierung mit einer komplementären Ziel-Nukleinsäuresequenz eignet und bei dem diese Nukleinsäure durch direkte chemische Brückenbildung eines Erkennungssystem, insbesondere eines Erkennungsenzyms, markiert wird, um später den so gebildeten Hybriden nachweisen zu können, dadurch gekennzeichnet, daß man die Markierung durch direkte chemische Brückenbildung des Erkennungssystems an den Guaningruppen der Sonden-Nukleinsäure mit Hilfe einer aus 4-Azidophenylglyoxal (APG) und Kethoxal oder deren Derivaten ausgewählten heterobifunktionellen Verbindung durchführt und daß es sich bei dieser Nukleinsäure der Sonde um eine modifizierte Einzelstrang-Nukleinsäure handelt, die von sich aus nicht zur Hybridisierung mit der Ziel-Nukleinsäure fähig ist und an die zur Hybridisierung geeignete Sonden-Nukleinsäuresequenz durch kovalente chemische Bindung angeheftet wird.

2. Verfahren nach Anspruch 1, dadurch gekennzeichnet, daß man zuerst die heterobifunktionelle Verbindung im Dunklen mit der zu verbrückenden Nukleinsäure reagieren läßt, um so eine kovalente Bindung zwischen der heterobifunktionellen Verbindung und den Guaningruppen der Nukleinsäure zu bilden, und dadurch, daß man das Ganze anschließend unter Lichtaktivierung mit einem Erkennungssystem, das sich zur Bildung einer Bindung mit dem aus der Azidfunktion durch Lichtaktivierung entstandenen Nitren eignet, reagieren läßt.

3. Verfahren nach einem der Ansprüche 1 und 2, dadurch gekennzeichnet, daß es sich bei der an die Sonde angeheftete Nukleinsäure um eine nichtkodierende natürliche oder synthetische Nukleinsäure aus der DNA, RNA, poly-A-G oder poly-dA-dG umfassenden Gruppe oder um eine natürliche Nukleinsäure, deren Sequenz so gewählt wird, daß sie während der Hybridisierung die Ziel-Nukleinsäuresequenz nicht ungünstig beeinflußt, handelt.

4. Verfahren nach Anspruch 3, dadurch gekennzeichnet, daß man die Reaktion der Brückenbildung an einer zuvordenaturierten Einzelstrang-Nukleinsäure durchführt.

5. Verfahren nach einem der Ansprüche 3 und 4, dadurch gekennzeichnet, daß man die verbrückte Nukleinsäure an die Sonde chemisch anheftet.

6. Verfahren nach einem der Ansprüche 1 und 2, dadurch gekennzeichnet, daß es sich bei dieser markierten Nukleinsäure um einen Einzelstrang-Vektor mit einer zirkulären Einzelstrang-Nukleinsäuresequenz handelt.

7. Verfahren nach Anspruch 6, dadurch gekennzeichnet, daß es sich bei dem Einzelstrang-Vektor um eine Phagen-, Virus-, Plasmid- oder auch Episom-Nukleinsäure handelt.

8. Verfahren nach Anspruch 7, dadurch gekennzeichnet, daß es sich bei dem Einzelstrang-Vektor um einen M13-Phagen handelt.

9. Verfahren nach einem der Ansprüche 6 bis 8, dadurch gekennzeichnet, daß der Vektor die Sonden-Nukleinsäuresequenz in seine zirkuläre Sequenz einkloniert enthält.

**10.** Verfahren nach einem der Ansprüche 1 bis 9, dadurch gekennzeichnet, daß es sich bei dem Erkennungssystem um ein insbesondere aus der Gruppe der Enzyme, vorzugsweise alkalischer Phosphatase, der Antikörper usw. ausgewähltes Protein handelt.

**11.** Verfahren nach Anspruch 10, dadurch gekennzeichnet, daß man anschließend am Protein einen Protein-, Enzym- oder Multienzymkomplex bildet, der sich zur Amplifizierung des Nachweissignals eignet, wobei die Komplexbildung nach bekannten Verfahren zur Bildung kovalenter Protein-Protein-Bindungen, insbesondere mit Hilfe von Glutaraldehyd oder EEDC, durchgeführt wird.

**12.** Verfahren nach einem der Ansprüche 1 bis 9, dadurch gekennzeichnet, daß das Erkennungssystem nicht proteinartig ist und insbesondere aus der Gruppe der fluoreszierenden und phosphoreszierenden Verbindungen ausgewählt wird oder auch ein Biolumineszenzsystem ist, das sich zur Umwandlung eines nichtlumineszierenden Substrats in ein lumineszierendes Produkt eignet.

**13.** Reagenzkit zur Ausführung des Verfahrens nach einem der Ansprüche 1 bis 12, dadurch gekennzeichnet, daß es folgendes umfaßt:

- eine Flasche mit Nukleinsäuren, die mit einem Nachweissystem verbrückt sind, zum Beispiel einem Nachweisenzym;
- eine Flasche mit einem Ligationssystem;
- eine Flasche mit einer gepufferten Reaktionsmischung, die zum Mischen mit Substraten geeignet ist (Sonden-Nukleinsäure und mit dem Nachweissystem verbrückte Nukleinsäure) ;
- eine Flasche mit einer Pufferlösung, die sich zum Mischen mit mindestens einem chromogenen Substrat eignet, das sich zur Reaktion mit dem Erkennungssystem eignet; sowie
- eine Flasche mit mindestens einem chromogenen Substrat.

**14.** Reaktionskit zur Ausführung des Verfahrens nach einem der Ansprüche 1 bis 12, dadurch gekennzeichnet, daß es im wesentlichen die folgenden Bestandteile umfaßt:

- 1 Flasche mit Luciferin,
- 1 Flasche mit ATP und einem Reaktionspuffer, sowie
- 1 Flasche mit Luciferase,

  oder
- 1 Flasche mit einer langkettigen Aldehydverbindung,
- 1 Flasche mit NAD(P)H + FMN + $H^+$ + einem Reaktionspuffer,
- 1 Flasche mit einer Diaphorase sowie
- 1 Flasche mit bakterieller Luciferase,

  oder
- 1 Flasche mit einer langkettigen Aldehydverbindung,
- 1 Flasche mit $FMNH_2$ und einem Reaktionspuffer, sowie
- 1 Flasche mit bakterieller Luciferase.

## Claims

**1.** Process for marking a nucleic acid in a nucleic probe comprising a nucleic-probe sequence able to hybridize with a complementary nucleic-target sequence, and in which the said nucleic acid is marked by direct chemical bridging with a tracer system, in particular a tracer enzyme, to enable subsequent detection to be made of the hybrid thus formed, characterized in that marking by direct chemical bridging is carried out with the tracer system on the guanine groups of the nucleic acid of the probe by means of a heterobifunctional compound selected from 4-azidophenyl-glyoxal (APG) and kethoxal or their derivatives and in that the said nucleic acid of the probe is a modified single-strand nucleic acid which is incapable on its own of hybridizing with the nucleic-target sequence and which is coupled with the nucleic-probe sequence able to hybridize by covalent chemical bonding.

**2.** Process according to claim 1 characterized in that the heterobifunctional compound is first of all reacted, in darkness, with the nucleic acid to be bridged so as to form a covalent bond between the heterobifunctional compound and

the guanine groups of the nucleic acid and in that the whole is then reacted together, without photoactivation, with a tracer system able to form a bond with the nitrene resulting from the photoactivation of the azide functional group.

**3.** Process according to either of claims 1 or 2, characterized in that the nucleic acid coupled with the probe is a natural or synthetic, non coding nucleic acid, selected from DNA, RNA, poly-A-G, poly-dA-dG or a natural nucleic acid the sequence of which is selected so as not to interfere with the nucleic-target sequence during hybridization.

**4.** Process according to claim 3 characterized in that the bridging reaction is carried out on a previously denatured nucleic acid, of the single strand type.

**5.** Process according to either of claims 3 or 4, characterized in that the bridged nucleic acid is coupled with the probe by chemical means.

**6.** Process according to either of claims 1 or 2, characterized in that the said marked nucleic acid is a single strand vector comprising a circular single strand nucleic sequence.

**7.** Process according to claim 6 characterized in that the single strand vector is a nucleic acid of a phage, a virus, a plasmid or an episome.

**8.** Process according to claim 7 characterized in that the single strand vector is a phage of the M13 type.

**9.** Process according to one of claims 6 to 8, characterized in that the vector includes, cloned in its circular sequence, the nucleic-probe sequence.

**10.** Process according to one of claims 1 to 9, characterized in that the tracer system is a protein selected in particular from enzymes, in particular alkaline phosphatase, antibodies, etc.

**11.** Process according to claim 10 characterized in that a proteic, enzymatic, or multi-enzymatic complex able to amplify the detection signal, is then formed on the protein, the formation of the complex being carried out by known methods, in particular by means of glutaraldehyde or EEDQ to form protein-protein covalent bonds.

**12.** Process according to one of claims 1 to 9, characterized in that the tracer system has a non proteic nature and is selected in particular from fluorescent compounds, phosphorescent compounds, or a bio-luminescence system able to transform a non luminescent substrate into a luminescent product.

**13.** Set of reagents for applying the process according to one of claims 1 to 12, characterized in that it comprises :

- a bottle of nucleic acids bridged with a detection system, for example, a detection enzyme;
- a bottle of a ligating system;
- a bottle of a buffered reaction mixture able to be mixed with substrates (nucleic acid-probe and nucleic acid bridged to the detection system) ;
- a bottle of a buffer solution able to be mixed with at least one chromogenic substrate able to react with the tracer system; and
- a bottle of at least one chromogenic substrate.

**14.** Set of reagents for applying the process according to one of claims 1 to 12, characterized in that it comprises essentially the following components :

- 1 bottle containing luciferin,
- 1 bottle containing ATP and a reaction buffer
- 1 bottle containing luciferase

or

- 1 bottle containing a long chain aldehydic compound,
- 1 bottle containing NAD(P)H + FMN + $H^+$ + a reaction buffer

- 1 bottle containing a diaphorase
- 1 bottle containing bacterial luciferase

or

- 1 bottle containing a long chain aldehydic compound
- 1 bottle containing $FMNH_2$ and a reaction buffer
- 1 bottle containing bacterial luciferase.

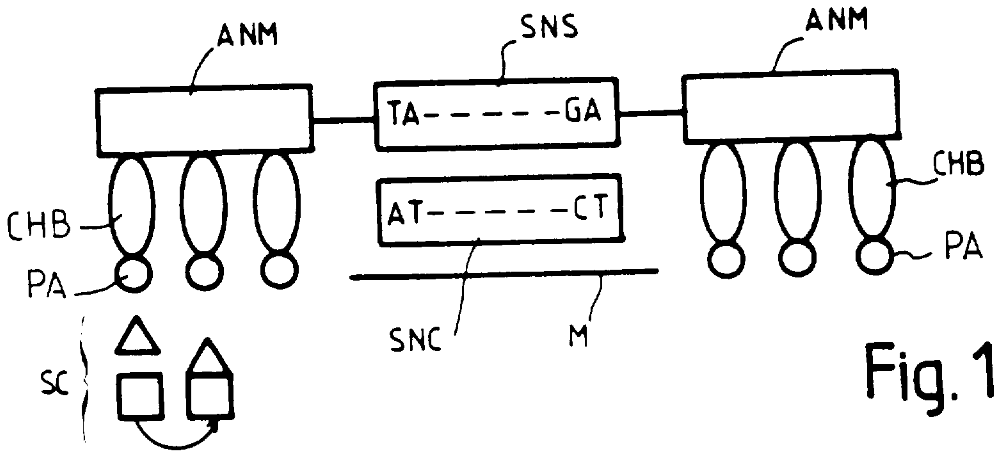


Fig. 1

Fig. 2

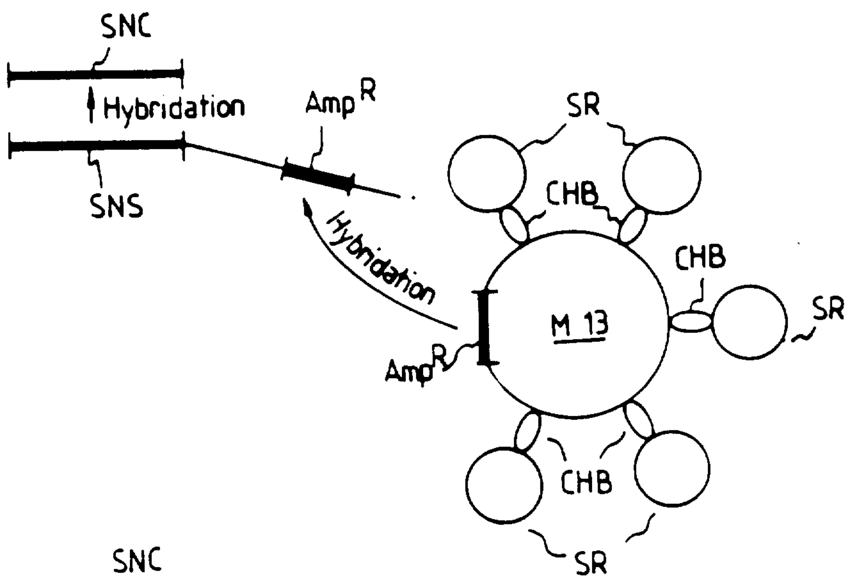


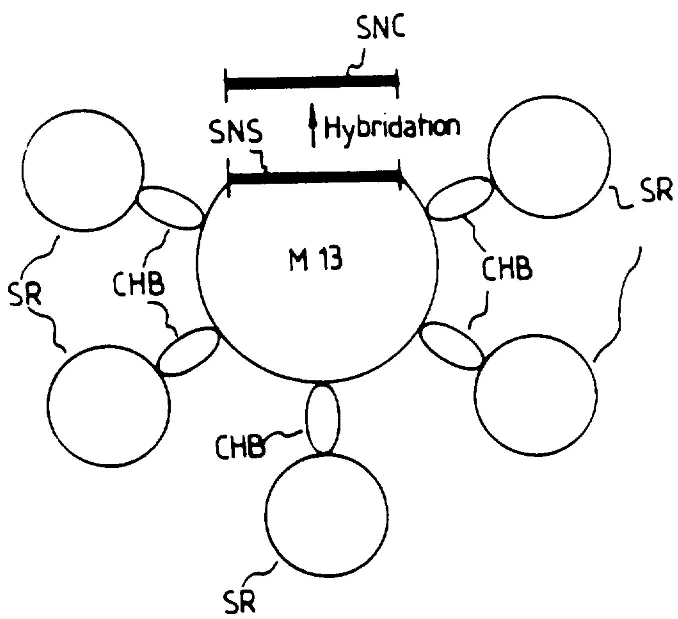


Fig.3

Fig. 4A

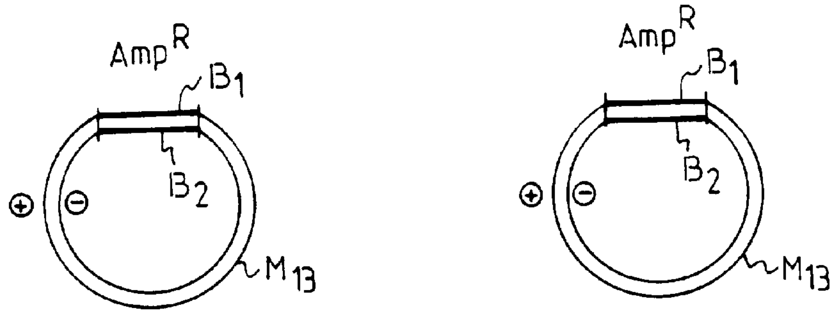


Fig. 4B

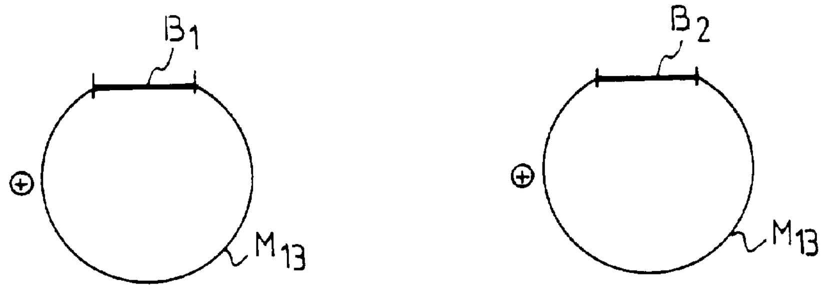


Fig. 4C

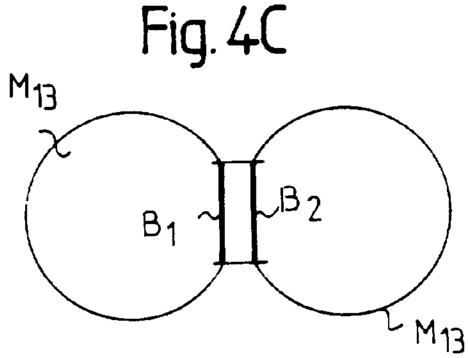


Fig. 4D

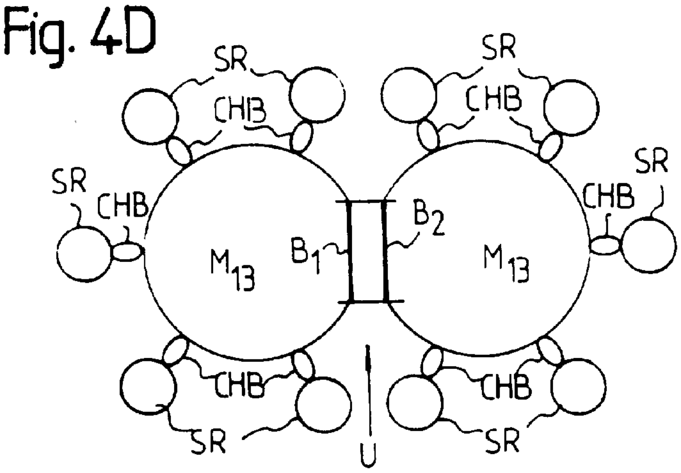


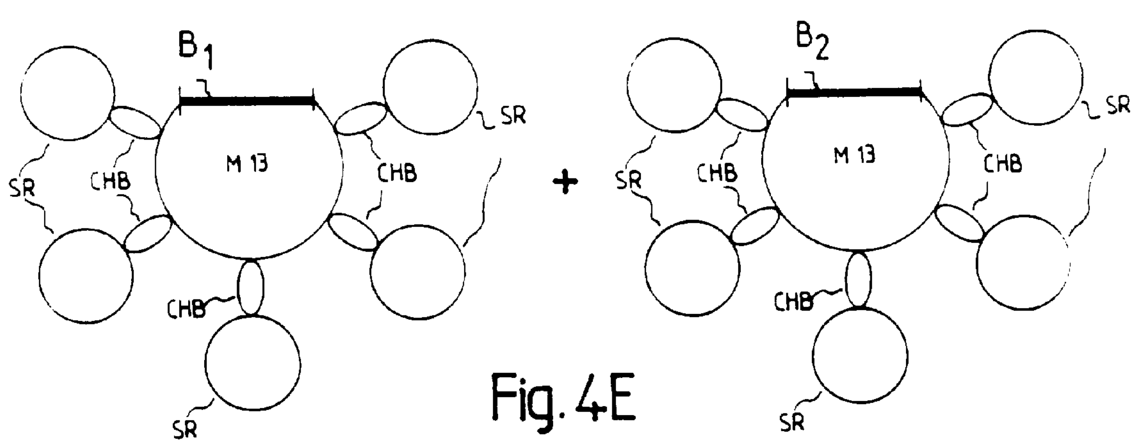


Fig. 4E